# EUROPÄISCHE PATENTSCHRIFT

(11) **EP 0 727 962 B1**
(45) Veröffentlichungstag und Bekanntmachung des Hinweises auf die Patenterteilung: **26.01.2000**
(21) Anmeldenummer: 94931501.4
(22) Anmeldetag: 28.10.1994
(51) Int. Cl.: A61B 7/00, A61B 5/08

(54) **VERFAHREN UND VORRICHTUNG ZUR AUTOMATISCHEN ERFASSUNG AUFFÄLLIGER ATEMGERÄUSCHE**
PROCESS AND DEVICE FOR THE AUTOMATIC DETECTION OF ABNORMAL BREATHING SOUNDS
PROCEDE ET DISPOSITIF DE DETECTION AUTOMATIQUE DE BRUITS RESPIRATOIRES ANORMAUX

(30) Priorität: 11.11.1993 DE 4338466
(43) Veröffentlichungstag der Anmeldung: 28.08.1996
(73) Patentinhaber: FRAUNHOFER-GESELLSCHAFT ZUR FÖRDERUNG DER ANGEWANDTEN FORSCHUNG E.V., 80636 München (DE); UFZ-UMWELTFORSCHUNGSZENTRUM Leipzig-Halle GmbH, 04318 Leipzig (DE)
(72) Erfinder: OBERST, Eberhard, D-01187 Dresden (DE); BECKER, Hans, D-01129 Dresden (DE); KRUMBIEGEL, Peter, D-04257 Leipzig (DE); HERBARTH, Olf, D-04275 Leipzig (DE)
(74) Vertreter: Gagel, Roland, Dr.
(86) Internationale Anmeldenummer: DE9401282
(87) Internationale Veröffentlichungsnummer: WO9513019

(56) Entgegenhaltungen:
- EP-A- 0 371 424
- EP-A- 0 504 945
- WO-A-91/03981
- GB-A- 2 240 392
- US-A- 5 133 346

## Beschreibung

### Technisches Gebiet

Die Erfindung bezieht sich auf ein Verfahren und eine Vorrichtung zur automatischen Erfassung und Überwachung auffälliger Atemgeräusche, insbesondere von bronchitis- oder asthmakranken und -gefährdeten Personen, über einen längeren Zeitraum.

Die nichtinvasive, nichtbelastende Erhebung experimenteller Meßgrößen, durch die bestimmte schädigende Umwelteinwirkungen auf Menschen objektiviert werden können, bereitet besonders in der Humanexpositionsforschung nach wie vor Probleme. Während für Parameter wie Puls und Blutdruck und selbst für die Entgiftungskapazität der Leber nicht belastende Methoden existieren, gibt es bis heute noch keine hinreichend einfache und belastungsfreie Methode, die eine objektive Aufzeichnung von typischen Atemgeräuschen im Zusammenhang mit ebenfalls objektiv gemessenen Immissionsgrößen erlaubt. Erst recht fehlt die Möglichkeit, solche Werte kontinuierlich und unter normalen Lebensbedingungen (beim Kind z. B. während des Spielens im Kindergarten oder im Freien, auf dem Schulweg usw.) entlang einer Zeitkoordinate zu registrieren und sofort oder später auszuwerten und gegebenenfalls ein Schwellwertsignal auszulösen.

### Stand der Technik

Aus der WO 90/04945 ist z. B. ein Diagnoseverfahren für Atemwegserkrankungen bekannt, bei dem zunächst vorzugsweise das Ausatmen der zu untersuchenden Person über ein Mikrofon aufgezeichnet wird.
In einem nächsten Schritt wird das aufgezeichnete Atemgeräusch einer Spektralanalyse unterzogen und das Ergebnis in einem Frequenz-Zeit-Diagramm dargestellt, wobei unterschiedlichen Intensitäten z. B. unterschiedliche Farben im Diagramm entsprechen. Diese Darstellungsweise soll den Arzt bei der Stellung der Diagnose unterstützen, da er hier eine zusätzliche optische Anzeige der Atemfrequenzen erhalt, aus deren Zeit- und Amplitudenverteilung ein krankhaftes Atemgeräusch zu erkennen ist.
Eine kontinuierliche Aufzeichnung unter normalen Lebensbedingungen ist jedoch hiermit nicht möglich, da die Fülle der Daten deren Auswertung und Speicherung sehr aufwendig macht. Die Auswertung muß hier optisch durch den behandelnden Arzt erfolgen.

In der WO 91/03981 ist ein Verfahren zur automatischen Erfassung krankhafter Atemgeräusche beschrieben, bei dem das Atemgeräusch einer zu überwachenden Person in einem zusammenhängenden Frequenzbereich in Echtzeit automatisch erfaßt, aufbereitet und ausgewertet wird, wobei die Schallwellen in ihrer Amplitude und Dauer mit für krankhafte Atemgeräusche typischen voreingestellten Werten verglichen werden und so das Auftreten eines krankhaften Atemgeräusches erkannt wird.
Auch dieses Verfahren ist als Diagnoseverfahren für das Labor konzipiert und somit nicht auf einfache Weise zur Erfassung und Aufzeichnung auffälliger Atemgeräusche unter normalen Lebensbedingungen geeignet. Die von Person zu Person unterschiedlichen charakteristischen Anzeichen für das Auftreten krankhafter Atemgeräusche finden keine Berücksichtigung, so daß nicht in jedem Fall eine zuverlässige Aussage über die Krankheitssituation möglich ist.

Aus GB-A-2 240 392 ist ein Verfahren bekannt , bei dem 2048 mal pro Sekunde ein Vergleich (pattern matching operations) zwischen einem im voraus gespeicherten kompletten Satz von den harmonischen Frequenzen (Fingerprint) der Atemgeräusche und später aufgenommenen spektrumstransformierten (harmonic content of the sounds) Atemgeräuschen gemacht wird. Eine Einschränkung des Vergleiches auf einzelne dieser harmonischen Frequenzen wird jedoch nicht vorgeschlagen.

### Darstellung der Erfindung

Der vorliegenden Erfindung liegt die Aufgabe zugrunde, ein Verfahren und eine Vorrichtung zur automatischen Erfassung auffälliger Atemgeräusche anzugeben, die die Datenmenge bei der Auswertung bei gleichzeitiger Erhöhung Zuverlässigkeit erheblich verringern, so daß bei entsprechender Miniaturisierung der Vorrichtung eine kontinuierliche und belastungsfreie Registrierung auffälliger Atemgeräusche entlang einer Zeitkoordinate über einen Zeitraum von Stunden oder Tagen unter normalen Lebensbedingungen möglich ist.

Die Aufgabe wird erfindungsgemäß mit dem Verfahren gemäß Anspruch 1 und der Vorrichtung gemäß Anspruch 15 gelöst.

Die Atemgeräusche der zu überwachenden Person werden, z. B. über ein Mikrofon, in Echtzeit erfaßt, aufbereitet und ausgewertet. Die Aufbereitung umfaßt z. B. Verstärkung, Rauschunterdrückung und Filterung. Bei der Auswertung werden nur die für die zu überwachende Person bereits vor Beginn der Messung individuell ermittelten charakteristische(n) Frequenz(en) berücksichtigt, an deren Position im Frequenzspektrum der Atemgeräusche der zu überwachenden Person ein auffälliges Atemgeräusch durch ein signifikantes Merkmal zu erkennen ist. Dieses signifikante Merkmal kann z. B. eine besonders hohe Amplitude an dieser Frequenzposition sein, die bei dieser Person nur im Falle eines krankhaften Ausbruches auftritt. Da die charakteristischen Frequenzen für jedes Individuum unterschiedlich ausfallen könnten, ist es wichtig, diese individuell für jede zu überwachende Person vorher zu bestimmen. Dies kann z. B. wie in Anspruch 12 oder 13 angegeben erfolgen.
Die an diesen charakteristischen Frequenzpositionen erfaßten Daten werden mit für die zu überwachende Person individuell ermittelten Daten auf Erfüllung eines oder mehrerer Kriterien für das Auftreten eines auffälligen Atemgeräusches verglichen. Diese individuell ermittelten Daten können z. B. Amplitudenwerte sein, deren Über- oder Unterschreitung bei dieser Person ein Anzeichen (Kriterium) für ein auffälliges Atemgeräusch sind. Die Bereitstellung der Meßinformation erfolgt entweder bei jedem Vergleich durch ein das Ergebnis des Vergleichs repräsentierendes Signal oder nur bei Erfüllung des/der Kriterien für das Auftreten eines auffälligen Atemgeräusches durch ein entsprechendes Signal. Das Signal kann z. B. in analoger Form durch einen bestimmten Spannungswert oder auch in digitaler Form bereitgestellt werden.
Die Reduktion der Auswertung auf diese charakteristische(n) Frequenz(en) führt zu einer erheblichen Datenreduktion und Verringerung des Rechenaufwandes und somit zu einer verbesserten Auswertemöglichkeit in Echtzeit.
Der Vergleich der Meßdaten mit für jede Person individuell ermittelten Vergleichswerten, wie auch die individuell ermittelten charakteristischen Frequenzen, erhöhen die Zuverlässigkeit des Verfahrens.
Durch die erhebliche Datenreduktion - im Minimalfall werden z. B. die Daten an nur einer charakteristischen Frequenzposition mit nur einem individuell bestimmten Amplitudenwert verglichen - wird auch der apparative Aufwand stark verringert, so daß es möglich ist, eine entsprechende Meßvorrichtung in miniaturisierter Bauweise (Mikrosystem) auszuführen (vgl. Anspruch 16), die dann über längere Zeit am Körper getragen werden kann, ohne die Bewegungsfreiheit des Trägers einzuschränken. Es wird somit eine kontinuierliche und belastungsfreie Registrierung auffälliger Atemgeräusche über einen Zeitraum von Stunden oder Tagen unter normalen Lebensbedingungen möglich.

Eine Meßvorrichtung zur Durchführung des eben beschriebenen Verfahrens besteht gemäß Anspruch 15 aus mindestens einem Mikrofon zum Erfassen des Atemgeräusches, mindestens einer Signalverarbeitungseinheit, die einen oder mehrere einstellbare Frequenzfilter zur Selektion der charakteristischen Frequenz(en) enthält, einer Auswerteeinheit, in der selektierte Meßdaten mit voreinstellbaren Vergleichswerten verglichen werden, einer Uhr, mit der das zeitliche Auftreten auffälliger Atemgeräusche festgestellt werden kann, einer Spannungsversorgung (z. B. Batterie), einer Speichereinheit zum Abspeichern der Vergleichswerte und der Vergleichsergebnisse, sowie einer Steuereinheit.
Es können auch mehrere Mikrofone mit nachgeschalteter Signalverarbeitungseinheit an der Körperoberfläche der zu überwachenden Person (vorzugsweise im Bereich der Lunge) angebracht werden, deren Daten jedoch einer gemeinsamen Auswerteeinheit zugeführt werden. Die Signalverarbeitungseinheit dient der Verstärkung, eventuell der Digitalisierung, der Rauschunterdrückung und der Filterung der eingehenden Signale, wobei durch einstellbare Frequenzfilter (analoger oder digitalerArt) die charakteristische(n) Frequenz(en) herausgefiltert werden. Die Vergleichswerte und die Vergleichsergebnisse, d. h. die das Ergebnis der Vergleiche repräsentierenden Signale oder die nur bei Erfüllung gewisser Vergleichskriterien bereitgestellten Signale, werden jeweils in einer Speichereinheit abgelegt, wobei mit jedem Signalwert eine durch die Uhr vorgegebene Zeitinformation abgespeichert werden kann. Nach dem Ende der Meßzeit (mehrere Stunden oder Tage ) kann die im Speicher abgelegte Information extern gelesen und somit in Verbindung mit der abgespeicherten Zeitinformation ein Rückschluß auf das zeitliche Auftreten auffälliger Atemgeräusche der überwachten Person gezogen werden.

Mit der Vorrichtung können charakteristische Geräusche im Atemweg der überwachten Personen als typische Merkmale im Zeit- und Frequenzbereich erkannt, von externen und nicht relevanten Schallereignissen isoliert und als objektive Parameter erfaßt werden. Diese für den Betroffenen individuellen, objektiven Parameter lassen sich mit ihrem Wert und ihrer Änderungstendenz zeitbezogen speichern.

Die Vorrichtung wird vorzugsweise als Minisensor (vgl. Anspruch 16) am Körper der Personen wenig belastend für längere Zeit getragen und dann extern gelesen. Damit lassen sich Korrelationen zu extern gemessenen Parametern (z. B. lufthygienische Daten) herstellen und nötigenfalls geeignete medizinische Behandlungen einleiten.
Insgesamt ergibt sich durch diese Erfindung eine den Probanden wenig belastende Methode zur Erfassung, Registrierung und Auswertung von Luftschadstoffwirkungen auf den Atemtrakt.

Ein solcher intelligenter monolithischer Bronchitissensor ist insbesondere geeignet zur automatischen Überwachung der Atemwege von bronchitis- bzw. asthmakranken und -gefährdeten Personen; als Frühwarngerät über einen bevorstehenden Anfall für Risikogruppen, das ständig oder bei bestimmten Wetter- oder Schadstofflagen mitgeführt bzw. am Körper getragen werden kann; für experimentell-epidemiologische Untersuchungen zur Incidenz von chronischer Bronchitis und Asthma bronchiale in Smogsituationen und in Abhängigkeit von der Konzentration von Luftschadstoffen im Lebensraum von Kindern; zur Senkung des Verbrauchs an Medikamenten und damit auch zur Verringerung von Risiken und Nebenwirkungen durch diese.

Besondere Ausführungsformen der Erfindung sind in den Unteransprüchen angegeben.
In einer vorteilhaften Ausgestaltung wird gemäß Anspruch 2 bei jedem Vergleich ein das Ergebnis des Vergleichs repräsentierender Wert gespeichert. Dieser Wert läßt eine Aussage darüber zu, ob der jeweilige Meßwert größer, gleich oder kleiner als der Vergleichswert war. Durch die Speicherung der Vergleichsergebnisse ist es möglich, nach Ablauf einer Meßzeit von mehreren Stunden oder Tagen aus den gespeicherten Werten eine Information über die Häufigkeit, den Zeitpunkt und den Zeitverlauf des Auftretens auffälliger Atemgeräusche der untersuchten Person zu gewinnen. Natürlich ist es auch möglich, zusätzlich bei jedem Vergleich eine exakte Zeitinformation abzuspeichern.

Eine vorteilhafte Verringerung des Speicheraufwandes ergibt sich, wenn, wie in Anspruch 3 angegeben, nur bei Erfüllung des Kriteriums (oder der Kriterien) für das Auftreten eines auffälligen Atemgeräusches der zu untersuchenden Person ein bestimmter Wert zusammen mit einer Zeitinformation gespeichert wird. Es ist in diesem Falle natürlich auch möglich, nur die Zeitinformation (als das Ereignis repräsentierender Wert) zu speichern, so daß jede gespeicherte Zeitinformation ein Indiz für das Auftreten eines auffälligen Atemgeräusches zu dem jeweiligen Zeitpunkt ist. Die Zeitinformation kann eine absolute oder relative Zeitangabe darstellen, die es ermöglicht auf den absoluten Zeitpunkt rückzuschließen. Somit läßt sich nach Ablauf der Meßzeit feststellen, ob und zu welcher Zeit auffällige Atemgeräusche aufgetreten sind.

Anspruch 4 gibt eine besondere Ausführungsform des erfindungsgemäßen Verfahrens an, bei dem bei Erfüllung des/der Kriterien für ein auffälliges Atemgeräusch ein akustisches und/oder optisches und/oder elektrisches und/oder mechanisches Warnsignal ausgelöst wird. Es kann sich dabei z. B. um einem Wecksignal entsprechende Schallsignale, um das Blinken einer Leuchtdiode oder auch um einen Körperreiz auslösende Spannungsimpulse oder mechanische (taktile) Reize auf der Haut handeln, die durch eine entsprechende Signaleinheit (Anspruch 17) abgegeben werden. Auch eine Kombination der unterschiedlichen Möglichkeiten kann eingesetzt werden. Das entsprechende Signal sollte von der zu untersuchenden Person in jedem Fall ohne Verzögerung deutlich wahrgenommen werden können, so daß diese Person damit vor einem bevorstehenden Anfall gewarnt werden kann.

Da die auffälligen Atemgeräusche nur während des Ein- bzw. Ausatmens auftreten, werden bei der Ausführungsform des erfindungsgemäßen Verfahrens nach Anspruch 5 die Erfassung, Aufbereitung und Auswertung der Atemgeräusche auf die Ein- und Ausatemvorgänge beschränkt. Damit kann bei einer entsprechenden Vorrichtung, wie sie z. B. in Anspruch 18 beschrieben ist, der Energieverbrauch während der Meßzeit verringert werden. Dies wird in Anspruch 18 durch eine Stand-by-Schaltung erreicht, die in der Zeit zwischen zwei Atemzyklen die Vorrichtung in den sog. Schlafmodus schaltet.

Vorzugsweise wird gemäß Anspruch 6 die Atemfrequenz der zu überwachenden Person zunächst fest eingestellt. Eine Veränderung der Atemfrequenz während der Messung (z. B. verursacht durch erhöhte Anstrengungen der Person) wird aufgrund der charakteristischen Geräusche des Ein- bzw. Ausatemvorgangs automatisch erkannt und der voreingestellte Wert der Atemfrequenz entsprechend angepaßt. Dies kann z. B. dergestalt erfolgen, daß zunächst die Erfassung der Atemgeräusche nur während durch die voreingestellte Atemfrequenz vorgegebenen festen Zeitfenstern erfolgt (Erfassung nur des Ein- und Ausatemvorgangs). Enden z. B. die für das Ausatmen charakteristischen Geräusche bereits zu einem früheren Zeitpunkt innerhalb des geöffneten Zeitfensters, so wird der Wert der eingestellten Atemfrequenz entsprechend erhöht. In gleicher Weise wird der Wert der Atemfrequenz verringert, wenn der Einatemvorgang innerhalb des Zeitfensters erst zu einem späteren Zeitpunkt einsetzt. Auf diese Weise können die Pausen zwischen zwei Atemzyklen optimal zur Energieeinsparung ausgenutzt werden.

In der bevorzugten Ausführungsform nach Anspruch 7 wird die bei der Auswertung zu berücksichtigende charakteristische Frequenz (oder auch mehrere charakteristische Frequenzen) durch geeignete einstellbare Filter aus dem erfaßten Frequenzbereich herausgefiltert, so daß nur die Daten der interessierenden Frequenzen der Auswertung zugeführt werden.

Gemäß Anspruch 8 wird als Kriterium für das Auftreten auffälliger Atemgeräusche die Überschreitung eines Amplitudenschwellwertes bei einer charakteristischen Frequenz gewählt. Der Amplitudenschwellwert (als Vergleichswert) entspricht einem für die zu untersuchende Person vor der Messung individuell ermittelten Wert, dessen Überschreitung bei dieser Person ein Anzeichen für das Auftreten eines auffälligen Atemgeräusches ist.

Die Ansprüche 9 und 10 geben besondere Ausführungsformen des erfindungsgemäßen Verfahrens an, bei denen die das Ergebnis der Vergleiche repräsentierenden Werte in einer Form gespeichert werden, in der sie eine Information über die Amplitude des jeweiligen Meßwertes bei der jeweiligen Frequenz oder auch über die Summe der Amplitudenwerte bestimmter charakteristischer Frequenzen enthatten. Bei beiden Ausführungsformen kann somit aus den gespeicherten Werten eine Information über die Intensität des jeweiligen Anfalls gezogen werden.

Das Auftreten starker Störgeräusche während der Meßzeit, die die Messung beeinträchtigen können, wird gemäß Anspruch 11 durch Überschreitung eines voreingestellten Amplitudenschwellwertes erkannt und die Signalverarbeitung in diesem Fall unterbrochen. Tritt das Störgeräusch in der Pause zwischen zwei Atemzyklen auf, wird die Erfassung und Auswertung beim darauf folgenden Ein- und Ausatemvorgang nicht durchgeführt, tritt das Störgeräusch während der Erfassung und Auswertung auf, wird die Auswertung für diesen Ein- und Ausatemvorgang abgebrochen und nicht berücksichtigt.

Die für die zu untersuchende Person charakteristische(n) Frequenz(en) und individuell ermittelten Daten (z. B. Amplitudenschwellwerte) werden in der Ausführungsform nach Anspruch 12 durch eine getrennte Aufzeichnung und Auswertung der Atemgeräusche dieser Person vor Beginn der Messung gewonnen. Dies kann z. B. mittels einer Auskultation durch einen Arzt erfolgen.

In der Ausführungsform nach Anspruch 13 werden vor Beginn der Messung zunächst aus dem Atemgeräusch der zu untersuchenden Person deren Normfrequenzen ermittelt. Über diese individuell gemessenen Normfrequenzen werden aus einem sogenannten Nomogramm die für diese Person zu erwartenden charakteristischen Frequenzen (Pathofrequenzen) bestimmt, die beim erfindungsgemäßen Verfahren berücksichtigt werden (vgl. Ausführungsbeispiel).

Gemäß Anspruch 14 werden die Atemgeräusche an einer oder mehreren Stellen der Körperoberfläche (z. B. an verschiedenen Stellen im Bereich der Lunge) der zu überwachenden Person erfaßt. Die an verschiedenen Stellen erfaßten Daten werden zusammengeführt, so daß die Auswertung und Speicherung gemeinsam erfolgen kann.

Bei der bevorzugten Ausführungsform der erfindungsgemäßen Vorrichtung nach Anspruch 16 ist diese in miniaturisierter Bauweise ausgeführt und auf einem oder mehreren Chips integriert. Damit ist es, wie bereits weiter oben beschrieben, möglich, die Vorrichtung als Sensor über einen größeren Zeitraum am Körper zu tragen, ohne in der Bewegungsfreiheit eingeschränkt zu sein. Eine Überwachung der Atemgeräusche der zu untersuchenden Person unter normalen Lebensbedingungen wird dadurch ermöglicht.

Das erfindungsgemäße Verfahren und die Vorrichtung zur Durchführung des Verfahrens werden nun anhand eines Ausführungsbeispiels in Zusammenhang mit der Zeichnung näher erläutert.

Dabei zeigt:

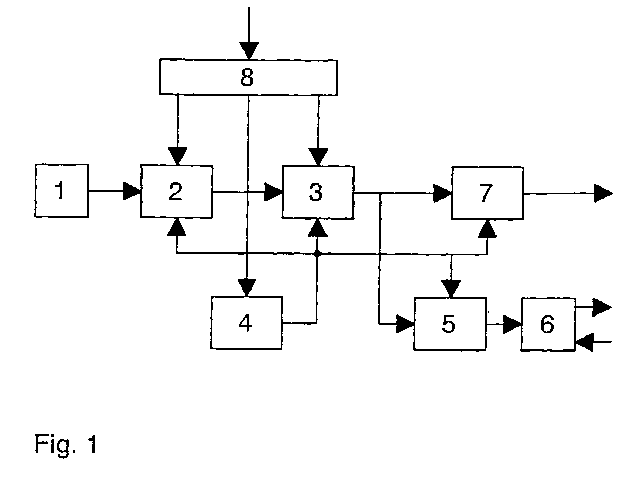

- Figur 1: ein Blockschaltbild eines Ausführungsbeispieles des erfindungsgemäßen Verfahrens.

### Ausführungsbeispiel

Durch das Mikrofon (1) werden die Atemgeräusche aufgenommen. Während in 2 eine Synchronisation auf die Atemfrequenz des Trägers erfolgt, werden gleichzeitig die vom Mikrofon (1) abgegebenen Signale durch Frequenz- und Amplitudenfilter darauf untersucht, ob sie Störgeräusche sind. In diesem Fall wird eine weitere Signalverarbeitung unterbunden. Nur Signale im interessierenden Frequenz- und Amplitudenbereich werden einer Merkmalsextraktion (3) zugeführt. Hier werden die Frequenz- und Amplitudensignale mit individuell einstellbaren Merkmalen verglichen und diese Vergleichsergebnisse gequantelt zur weiteren Bearbeitung bereitgestellt. In 7 erfolgt eine Wandlung z. B. in ein akustisches Signal, durch das der Träger des Bronchitissensors vor einem bevorstehenden Anfall rechtzeitig gewarnt wird. Außerdem werden die in 3 aufbereiteten Signale zur zeitgerechten Speicherung dem Speicher (5) zugeführt, in dem sie über einen längeren Zeitraum abgelegt werden können. Das Auslesen der gespeicherten Werte erfolgt dann seitens einer Zentrale über 6, z. B. durch eine Ankopplung an ein Telefon. Die Einheit 4 ist funktionell eine Uhr, die extern gesetzt werden kann, die Synchronisation mit der Atemfrequenz und damit die Störunterdrückung in 2 sowie die Merkmalsextraktion in 3 unterstützt und für die Abbildung der zu speichernden Werte auf eine kontinuierliche oder diskontinuierliche Zeitskala dient. Die Umsetzung der kompakten, externen Initialisierungs- und Steuerinformationen auf die Subsysteme des Sensorsystems erfolgt über die Steuereinheit 8.

Zunächst erfaßt der Arzt während einer Auskultation vor dem Anlegen des Sensors die pathologischen Bronchiengeräusche der zu kontrollierenden Person. Die dafür typischen Schallfrequenzen (Pathofrequenzen) werden extern aufgezeichnet. Nur diese charakteristischen Frequenzen und deren daraus ermittelte typische Merkmale werden dann dem Sensor als innerer Standard eingegeben und für die Signalerkennung zur Verfügung gestellt. Entlang einer Zeitachse tastet der Sensor nur diese charakteristischen Frequenzen ab. Sobald Interferenz mit den vorgegebenen Merkmalen auftritt, wird dieser Zustand als Signal registriert bzw. gespeichert. Die Uhrzeit und eventuell die Zeitdauer dieses Interferenzereignisses sind hinreichende Parameter für die automatische Überwachung der Atemwege bezüglich der in Frage stehenden pathologischen Zustände.

Bei Personen, wie z. B. potentiell gefährdeten Kindern, bei denen der vorab benötigte innere Standard des pathologischen Geräusches nicht individuell gewonnen werden kann, ist die jeweilige Pathofrequenz einer Eichkurve zu entnehmen, die zuvor auf statistischem Wege und unter Berücksichtigung der Größen- und Altersabhängigkeit erstellt wurde.
Für diesen Personenkreis wird zunächst das Frequenzspektrum der Normatemgeräusche individuell gewonnen. Für diese Frequenzen (z. B. für das Ausatmungsgeräusch) kann auch am zuverlässigsten und genauesten eine Eichkurve der alters- und größenabhängigen Normgeräuschsfrequenzen erstellt werden. Auch der charakteristische Abstand zwischen diesen Normfrequenzen und den erwarteten Pathofrequenzen kann statistisch ermittelt werden. Auf dieser Grundlage kann man mit der individuell gemessenen Normfrequenz in ein entsprechendes Nomogramm gehen, dort die für das Individuum erwartete Pathofrequenz ablesen und diese dem Sensor als inneren Standard vorgeben, das heißt, die Filter den charakteristischen Frequenzen entsprechend einstellen.

Der gesamte Sensor ist als Mikrosystem ausgebildet. Die Aufnahme des Geräusches erfolgt durch ein kapazitiv auslesbares Chipmikrofon (1). Der Aufnahmebereich beträgt z. B. 20 bis 2000 Hz, wodurch bereits bei der Aufnahme der interessierenden Signale eine erste Filterwirkung im System erreicht wird. Eine weitere individuelle Einstellung erfolgt durch nachgeschaltete programmierbare Filter (2).

Für derartige Mikrosysteme ist das Problem des Energieverbrauches relevant. Deshalb nimmt der Sensor nur während des Ein- und Ausatmens Information auf und verarbeitet diese. In der Zwischenzeit wird ein sogenannter Schlafmodus (Stand-by-Modus) gefahren, der Sensor bereitet sich auf die nächste Meßzeit vor.

Der Zustand der Atemwege der überwachten Person wird durch die Geräusche beim Ein- und Ausatmen charakterisiert. Die Atemzüge sind markant. Die Atemfrequenz kann bei Inbetriebnahme des Gerätes eingestellt werden. Sie entspricht dem Abstand zweier aufeinanderfolgender, relativ ähnlicher Signalgruppen (Atemimpulse gewonnen aus dem Frequenz/Amplitudenspektrum). Eine Nachführung der Änderung der Atemfrequenz (Veränderung des zeitlichen Abstandes zwischen zwei Atemzügen) wird aufgrund entsprechender Messungen automatisch durchgeführt (vgl. auch Anspruch 6).

Ein erstes Ausblenden oder Unterdrücken von Störgeräuschen erfolgt über die Einschränkung des beobachteten Frequenzbandes auf z. B. 20 bis 2000 Hz. Die Signalverarbeitung wird außerdem gesperrt, wenn zwischen zwei Atemimpulsen oder während der Atmung Signale auftreten, die einen eingestellten Schwellwert überschreiten und deshalb eine einwandfreie Erkennung und Auswertung der Atemzüge erschweren oder verhindern würden.

Die aus dem Frequenz/Amplitudenspektrum gewonnenen Daten werden, unter Umständen nach Anwendung geeigneter Transformationsalgorithmen (z. B. Laplace oder Fourier-Transformationen), mit den individuell ermittelten voreingestellten Daten verglichen. Bei Überschreitung individuell ermittelter eingestellter Werte wird der Zeitpunkt ihres Auftretens gespeichert. Dadurch wird die Registrierung bzw. Überwachung auf wenige Werte eingeschränkt, wodurch der Speicheraufwand und der Leistungsverbrauch des Sensors erheblich gesenkt werden können.

Das Einlesen in den Speicher erfolgt in der üblichen Weise: Ein Zähler zählt die belegten Speicherplätze bzw. gibt den nächsten Speicherplatz an. Das Auslesen erfolgt von außen gesteuert unter Nutzung des Zählers, der beim Auslesen zurückgesetzt bzw. auf Null gesetzt wird.

## Patentansprüche

1. Verfahren zur automatischen Erfassung auffälliger Atemgeräusche, insbesondere von bronchitis- oder asthmakranken und -gefährdeten Personen, bei dem die Atemgeräusche einer zu überwachenden Person in einem zusammenhängenden Frequenzbereich in Echtzeit automatisch erfaßt, aufbereitet und ausgewertet werden,
wobei
aus dem erfaßten Frequenzbereich bei der Auswertung eine oder mehrere vorher für die zu untersuchende Person individuell bestimmte charakteristische Frequenz (en) berücksichtigt werden, an deren Position im Frequenzspektrum des Atemgeräusches der zu überwachenden Person ein auffälliges Atemgeräusch durch ein signifikantes Merkmal zu erkennen ist,
bei der Auswertung nur die an diesen Frequenzpositionen erfaßten Daten mit für die zu überwachende Person individuell ermittelten Daten auf Erfüllung eines oder mehrerer Kriterien für das Auftreten eines auffälligen Atemgeräusches verglichen werden,
und entweder bei jedem Vergleich ein das Ergebnis des Vergleichs repräsentierendes Signal oder nur bei Erfüllung des/der Kriterien ein dieses Ereignis repräsentierendes Signal bereitgestellt und ein das Vergleichsergebnis repräsentierender Wert gespeichert werden.

2. Verfahren nach Anspruch 1,
**dadurch gekennzeichnet,**
daß bei jedem Vergleich ein das Ergebnis des Vergleichs repräsentierender Wert gespeichert wird,
so daß nach Ablauf einer Meßzeit von mehreren Stunden oder Tagen aus den gespeicherten Werten eine Information über die Häufigkeit und den Zeitverlauf des Auftretens auffälliger Atemgeräusche gewonnen werden kann.

3. Verfahren nach Anspruch 1,
**dadurch gekennzeichnet,**
daß nur bei Erfüllung des/der Kriterien ein dieses Ereignis repräsentierender Wert einschließlich einer Information, die einen Rückschluß auf das zeitliche Auftreten des Ereignisses zuläßt, gespeichert wird/werden, so daß nach Ablauf einer Meßzeit von mehreren Stunden oder Tagen aus den gespeicherten Werten eine Information über die Häufigkeit und den Zeitverlauf des Auftretens auffälliger Atemgeräusche gewonnen werden kann.

4. Verfahren nach einem der Ansprüche 1 bis 3,
**dadurch gekennzeichnet,**
daß bei Erfüllung des/der Kriterien für ein auffälliges Atemgeräusch ein akustisches und/oder optisches und/oder elektrisches und/oder mechanisches Warnsignal ausgelöst wird.

5. Verfahren nach einem der Ansprüche 1 bis 4,
**dadurch gekennzeichnet,**
daß die Erfassung, Aufbereitung und Auswertung nur während des Einund/oder Ausatmens erfolgt.

6. Verfahren nach Anspruch 5,
**dadurch gekennzeichnet,**
daß der Ein- und/oder Ausatemvorgang aufgrund der typischen Atemgeräusche erkannt und ein voreingestellter Wert des zeitlichen Abstandes der Atemzyklen, während dessen keine Erfassung, Aufbereitung und Auswertung erfolgt, bei Veränderung der Atemfrequenz selbständig angepaßt wird.

7. Verfahren nach einem der Ansprüche 1 bis 6,
**dadurch gekennzeichnet,**
daß die bei der Auswertung zu berücksichtigende(n) Frequenz(en) durch geeignete Filter aus dem erfaßten Frequenzbereich herausgefiltert werden.

8. Verfahren nach einem der Ansprüche 1 bis 7,
**dadurch gekennzeichnet,**
daß als ein Kriterium für das Auftreten auffälliger Atemgeräusche die Überschreitung eines Amplituden-Schwellwertes gewählt wird.

9. Verfahren nach einem der Ansprüche 2 bis 8,
**dadurch gekennzeichnet,**
daß die Werte in einer Form gespeichert werden, in der sie eine Information über den jeweils erfaßten Amplitudenwert bei der jeweiligen Frequenz enthalten.

10. Verfahren nach einem der Ansprüche 2 bis 8,
**dadurch gekennzeichnet,**
daß die Werte in einer Form gespeichert werden, in der sie eine Information über die Summe der Amplitudenwerte bestimmter charakteristischer Frequenzen enthalten.

11. Verfahren nach einem der Ansprüche 1 bis 10,
**dadurch gekennzeichnet,**
daß das Auftreten starker Störgeräusche während der Meßzeit durch Überschreitung eines voreingestellten Amplitudenschwellwertes erkannt und die Signalverarbeitung in diesem Fall unterbrochen wird.

12. Verfahren nach einem der Ansprüche 1 bis 11,
**dadurch gekennzeichnet,**
daß die für die zu untersuchende Person charakteristische(n) Frequenz(en) und individuell ermittelten Daten durch eine getrennte Aufzeichnung- und Auswertung der Atemgeräusche dieser Person vor Beginn der Messung gewonnen werden.

13. Verfahren nach einem der Ansprüche 1 bis 11,
**dadurch gekennzeichnet,**
daß die für die zu untersuchende Person charakteristische(n) Frequenz(en) vor Beginn der Messung aus einem Nomogramm gewonnen werden, nachdem aus dem Atemgeräusch der zu untersuchenden Person die Normfrequenzen bestimmt wurden.

14. Verfahren nach einem der Ansprüche 1 bis 13,
**dadurch gekennzeichnet,**
daß die Atemgeräusche an einer oder mehreren Stellen der Körperoberfläche der zu überwachenden Person erfaßt werden, und die erfaßten Daten gemeinsam ausgewertet und gespeichert werden.

15. Vorrichtung zur Durchführung des Verfahrens nach einem der Ansprüche 1 bis 14, bestehend aus
- mindestens einem Mikrofon (1) zum Erfassen des Atemgeräusches,
- mindestens einer Signalverarbeitungseinheit (2), die einen oder mehrere einstellbare Frequenzfilter zur Selektion voreinstellbarer charakteristischer Frequenzen enthält,
- einer Auswerteeinheit (3), die für den Vergleich selektierter, mit dem Mikrofon erfaßter Meßdaten mit voreinstellbaren Vergleichswerten ausgelegt ist, und entweder bei jedem Vergleich oder nur bei Erfüllung des/der Kriterien für das Auftreten eines auffälligen Atemgeräusches einen das Vergleichsergebnis repräsentierenden Wert abspeichert,
- einer Uhr (4), die so in die Vorrichtung integriert ist, daß deren jeweilige Zeitinformation in Abhängigkeit des Ergebnisses des Vergleichs gespeichert werden kann,
- einer Spannungsversorgung,
- einer Speichereinheit (5), zum Abspeichern der Vergleichswerte und Vergleichsergebnisse und
- einer Steuereinheit (8) zur Umsetzung kompakter, externer Initialisierungs- und Steuerinformationen auf die Subsysteme (2, 3, 4) der Vorrichtung,
- wobei die Vorrichtung so ausgestaltet ist, daß sie vom Probanden während der Messung über längere Zeit unter normalen Lebensbedingungen am Körper getragen werden kann, ohne dessen Bewegungsfreiheit einzuschränken.

16. Vorrichtung nach Anspruch 15,
**dadurch gekennzeichnet,**
daß sie in miniaturisierter Bauweise ausgeführt und auf einem oder mehreren Chips integriert ist.

17. Vorrichtung nach Anspruch 15 oder 16,
**dadurch gekennzeichnet,**
daß sie eine Signaleinheit aufweist, die bei Bedarf ein akustisches und/oder optisches und/oder elektrisches und/oder mechanisches Signal abgibt.

18. Vorrichtung nach einem der Ansprüche 15 bis 17,
**dadurch gekennzeichnet,**
daß sie eine Stand-By-Schaltung aufweist, mit der der Energieverbrauch der Vorrichtung in der Zeit zwischen zwei Atemzyklen reduzierbar ist.

## Claims

1. Method of automatically detecting abnormal respiratory sounds, particularly on persons suffering from or exposed to the risk of bronchitis or asthma, wherein the respiratory sounds of a person to be monitored are automatically detected, processed and evaluated in real time over a coherent frequency range,
wherein
one or several characteristic frequencies, which were previously determined on an individual basis for the person under examination, from the covered frequency range are considered in the evaluation, with an abnormal respiratory sounds being detectable by a significant feature at the position of these frequencies within the frequency spectrum of the respiratory sounds of the person to be monitored,
during evaluation only that data which is detected at these frequency positions is compared against the data established on an individual basis for the person to be monitored for establishing the fulfilment of one or several criteria of the occurrence of an abnormal respiratory sound,
and either for each comparison a signal representative of the result of the comparison or only when the criterion or criteria is/are satisfied a signal representative of this event is made available, and a value is stored which is representative of the result of the comparison.

2. Method according to Claim 1,
**characterised in**
that for each comparison a value representative of the comparison is stored so that upon expiration of a measuring period of several hours or days an information about the frequency and the development of the occurrence of abnormal respiratory sounds may be obtained from the stored values.

3. Method according to Claim 1,
**characterised in**
that only when said criterion/criteria is/are satisfied a value representative of this event is stored, including an information which permits a conclusion of the occurrence of the event in terms of time so that upon expiration of a measuring period of several hours or days an information about the frequency and the development of the occurrence of abnormal respiratory sounds may be obtained from the stored values.

4. Method according to any of the Claims 1 to 3,
**characterised in**
that when said criterion/criteria of an abnormal respiratory sound is/are satisfied an acoustic and/or optical and/or electrical and/or mechanical alarm signal is triggered.

5. Method according to any of the Claims 1 to 4,
**characterised in**
that the detection, processing and evaluation are performed only during inspiration and/or expiration.

6. Method according to Claim 5,
**characterised in**
that the inspiration and/or expiration process is recognised on the basis of the typical respiratory sounds and a pre-set value of the time intervals of the breathing cycles, during which detection, processing and evaluation is not performed, is automatically adapted when the respiratory frequency varies.

7. Method according to any of the Claims 1 to 6,
**characterised in**
that the frequency/frequencies to be considered during evaluation is/are filtered out from the covered frequency range by means of appropriate filters.

8. Method according to any of the Claims 1 to 7,
**characterised in**
that the event that an amplitude threshold is exceeded is selected as criterion of occurrence of abnormal respiratory sounds.

9. Method according to any of the Claims 2 to 8,
**characterised in**
that the values are stored in a form in which they contain an information about the respectively detected amplitude value at the respective frequency.

10. Method according to any of the Claims 2 to 8,
**characterised in**
that the values are stored in a form in which they contain an information about the total of the amplitude values of defined characteristic frequencies.

11. Method according to any of the Claims 1 to 10,
**characterised in**
that the occurrence of strong interference noise during the measuring period is detected by values exceeding a pre-set amplitude threshold and that the signal processing is interrupted in this case.

12. Method according to any of the Claims 1 to 11,
**characterised in**
that the frequency/frequencies characteristic of the person under examination and the data determined on an individual basis are obtained by a separate recording and evaluation of the respiratory sounds of this person prior to the beginning of the measurement.

13. Method according to any of the Claims 1 to 11,
**characterised in**
that the frequency/frequencies characteristic of the person under examination is/are obtained from a nomograph prior to the beginning of the measurement after the standard frequencies have been determined from the respiratory sounds of the person under examination.

14. Method according to any of the Claims 1 to 13,
**characterised in**
that the respiratory sounds are detected on one or several locations on the surface of the body of the person to be monitored, and that the data so detected is jointly evaluated and stored.

15. Apparatus for implementing the method according to any of the Claims 1 to 14, consisting of
- at least one microphone (1) for detecting the respiratory sounds,
- at least one signal processing unit (2) including one or several adjustable frequency filters for the selection of pre-settable characteristic frequencies,
- one evaluating unit (3) which is designed for the comparison of selected measuring data detected by means of the microphone against pre-settable reference values, and which stores a value representative of the result of the comparison only when said criterion/criteria of the occurrence of an abnormal respiratory sound is/are satisfied,
- a clock (4) so integrated into the apparatus that its respective information of time may be stored as a function of the result of the comparison,
- a power supply,
- a memory unit (5) for storing the reference values and the results of the comparison, and
- a controller (8) for converting compact external initialising and control information to the sub-systems (2, 3, 4) of the apparatus,
- wherein the apparatus is so configured that the test person can wear it on the body under normal living conditions during the measurement over a prolonged period of time without any restriction of his/her freedom of movement.

16. Apparatus according to Claim 15,
**characterised in**
that it is realised in a miniaturised structure and integrated on one or several chips.

17. Apparatus according to Claim 15 or 16,
**characterised in**
that it comprises a signalling unit which produces an acoustic and/or optical and/or electrical and/or mechanical signal whenever this is necessary.

18. Apparatus according to any of the Claims 15 to 17,
**characterised in**
that it comprises a stand-by circuit serving to reduce the power consumption of the apparatus in the interval between two breathing cycles.

## Revendications

1. Procédé à détecter, de façon automatique, du bruit respiratoire remarquable, en particulier dans des personnes qui souffre d'une bronchite ou de l'asthme ou qui sont menacées d'une telle maladie, dans lequel le bruit respiratoire d'une personne à surveiller est automatiquement détecté, traité et évalué en temps réel au-dedans d'une gamme de fréquences cohérente,
dans lequel
une ou plusieurs fréquences caractéristiques, qui étaient établies avant de façon individuelle pour la personne examinée, à partir de la gamme de fréquences détectée sont considérées dans l'évaluation, à un bruit respiratoire remarquable étant détectable par un attribut significatif à la position de ces fréquences au-dedans du spectre de fréquences du bruit respiratoire de la personne à surveiller,
pendant l'évaluation, seulement ces données, qui sont détectée à ces positions de fréquence, sont comparées aux données relevées individuellement pour la personne à surveiller afin d'établir un ou plusieurs critères satisfaits d'un son respiratoire remarquable,
et soit pour chaque comparaison un signal représentatif du résultat de la comparaison, soit seulement quand le critère(s) est/sont satisfait(s) un signal représentatif de cet événement est fourni, et une valeur est mise en mémoire qui est représentative du résultat de la comparaison.

2. Procédé selon la revendication 1,
**caractérisé en ce**
que pour chaque comparaison une valeur représentative de la comparaison est mise en mémoire
de façon qu'après l'expiration d'une période de mesure à plusieurs heures ou jours on peut obtenir, à la base des valeurs mises en mémoire, une information sur la fréquence et le développement de la présence du bruit respiratoire remarquable.

3. Procédé selon la revendication 1,
**caractérisé on ce**
qu'au seule cas où ledit critère/lesdits critères est/sont satisfait(s), une valeur représentative de cet événement est mise en mémoire, ci-inclus une information qui permet la déduction de la présence de événement par le temps, de façon qu'après l'expiration d'une période de mesure de quelques heures ou jours on puisse obtenir une information sur la fréquence et le développement de la présence d'un bruit respiratoire remarquable à la base des valeurs mises en mémoire.

4. Procédé selon une quelconque des revendications 1 à 3,
**caractérisé en ce**
qu'au cas où ledit critère/lesdits critères d'un son respiratoire remarquable est/sont satisfait(s), un signal d'alarme acoustique et/ou optique et/ou électrique et/ou mécanique est déclenché.

5. Procédé selon une quelconque des revendications 1 à 4,
**caractérisé en ce**
que la détection, le traitement et l'évaluation ne sont réalisés qu'au cours de l'inspiration et/ou de l'expiration.

6. Procédé selon la revendication 5,
**caractérisé en ce**
que le processus de l'inspiration et/ou de l'expiration est reconnu à la base du bruit respiratoire typique, et une valeur réglée à l'avance des intervalles des cycles de respiration, au cours desquels la détection, le traitement et l'évaluation ne sont pas réalisés, est automatiquement adaptée quand la fréquence de respiration subit une variation.

7. Procédé selon une quelconque des revendications 1 à 6,
**caractérisé en ce**
que la fréquence/les fréquences à considérer dans l'évaluation est/sont extraite(s) par filtrage de la gamme de fréquences détectée, moyennant des filtres appropriés.

8. Procédé selon une quelconque des revendications 1 à 7,
**caractérisé en ce**
que événement de dépassement d'un seuil d'amplitudes est choisi comme critère de la présence de bruit respiratoire remarquable.

9. Procédé selon une quelconque des revendications 2 à 8,
**caractérisé en ce**
que les valeurs sont mises en mémoire sous une forme dans laquelle elles renferment une information sur la valeur d'amplitude respectivement détectée à la fréquence respective.

10. Procédé selon une quelconque des revendications 2 à 8,
**caractérisé en ce**
que les valeurs sont mises en mémoire sous une forme dans laquelle elles renferment une information sur la somme des valeurs d'amplitude aux fréquences caractéristiques définies.

11. Procédé selon une quelconque des revendications 1 à 10,
**caractérisé en ce**
que la présence d'un brouillage fort au cours de la période de mesure est détectée par le dépassement d'un seuil d'amplitudes ajusté à l'avance, et en ce que dans cet cas le traitement des signaux est interrompu.

12. Procédé selon une quelconque des revendications 1 à 11,
**caractérisé en ce**
que la fréquence/les fréquences caractéristique(s) de la personne examinée et les données relevées individuellement sont obtenues par une opération séparée d'enregistrement et d'évaluation du bruit respiratoire de cette personne avant le début du mesurage.

13. Procédé selon une quelconque des revendications 1 à 11,
**caractérisé en ce**
que la fréquence/les fréquences caractéristique(s) de la personne examinée est/sont obtenues d'un nomogramme à alignement avant le début du mesurage, après l'établissement des fréquences normales à la base du bruit respiratoire de la personne examinée.

14. Procédé selon une quelconque des revendications 1 à 13,
**caractérisé en ce**
que le bruit respiratoire est détecté à un ou plusieurs endroits sur la surface du corps de la personne à surveiller, et en ce que les données relevées de cette façon sont évaluées et mises en mémoire en commun.

15. Appareil à réaliser le procédé selon une quelconque des revendications 1 à 14, composé des unités suivantes:
- au moins un microphone (1) à détecter le bruit respiratoire,
- au moins une unité de traitement de signaux (2), qui comprend un ou plusieurs filtres de fréquence ajustables pour la sélection des fréquences caractéristiques ajustables à l'avance,
- une unité d'évaluation (3), qui est conçue pour la comparaison des données de mesure choisies, qui sont détectées moyennant ledit microphone, avec des valeurs de référence ajustables à l'avance, et qui met en mémoire une valeur représentative du résultat de la comparaison seulement quand ledit critère/lesdits critères de la présence d'un son respiratoire remarquable est/sont satisfait(s),
- un compteur horloge (4) intégré dans l'appareil de façon qu'on puisse mettre en mémoire ses informations respectives sur le temps puis en fonction du résultat de la comparaison,
- un bloc d'alimentation,
- une unité de mémoire (5) à mémoriser les valeurs de référence et les résultats de ta comparaison, et
- un organe de commande (8) à convertir des informations compactes extérieurs d'initialisation et de commande et à les distribuer aux sous-systèmes (2, 3, 4) de l'appareil,
- dans lequel l'appareil est si configuré qu'un sujet d'expériences peut le porter sur le corps dans des conditions de vie au cours de la mesure assez longtemps, sans limitation de sa liberté de mouvement.

16. Appareil selon la revendication 15,
**caractérisé en ce**
qu'il est réalisé sous forme d'une structure miniaturisée, en étant intégré sur une ou plusieurs puces.

17. Appareil selon la revendication 15 ou 16,
**caractérisé en ce**
qu'il comprend une unité de signalisation, qui produit un signal acoustique et/ou optique et/ou électrique et/ou mécanique, quand cela sera nécessaire.

18. Appareil selon une quelconque des revendications 15 à 17,
**caractérisé en ce**
qu'il comprend un circuit de secours capable de réduire la consommation d'énergie de l'appareil dans l'intervalle entre deux cycles de respiration.
